# EUROPEAN PATENT SPECIFICATION

(11) **EP 4 434 502 B1**
(45) Date of publication and mention of the grant of the patent: **29.10.2025**
(21) Application number: 23164146.5
(22) Date of filing: 24.03.2023
(51) Int. Cl.: A61F 13/15

(54) **METHOD FOR MEASURING AND DETECTING WELDING PATTERNS AND CUT LOCATIONS ON A WEB OF ABSORBENT ARTICLES**
VERFAHREN ZUR MESSUNG UND ERKENNUNG VON SCHWEISSMUSTERN UND SCHNITTSTELLEN AUF EINER BAHN VON SAUGFÄHIGEN ARTIKELN
PROCÉDÉ DE MESURE ET DE DÉTECTION DE MOTIFS DE SOUDAGE ET D'EMPLACEMENTS DE DÉCOUPE SUR UNE BANDE D'ARTICLES ABSORBANTS

(43) Date of publication of application: 25.09.2024
(73) Proprietor: Ontex BV, 9255 Buggenhout (BE); Ontex Group NV, 9320 Erembodegem (BE)
(72) Inventor: Heege, Thomas, 56761 Düngenheim (DE); Torres Saavedra, Antonio, 56330 Kobern-Gondorf (DE)
(74) Representative: Saurat, Thibault

(56) References cited:
- US-A- 5 045 135
- US-A1- 2009 194 218

## Description

### TECHNICAL FIELD

The present disclosure refers to a method for manufacturing an absorbent hygiene article such as a diaper, a pant diaper or an incontinence guard comprising a plurality of components that are joined together during the manufacturing process and cut and separated into individual pieces. The disclosure provides a method for improved control of this severing step and the apparatus to carry out such method.

### BACKGROUND

Absorbent articles comprise a plurality of components, like web materials constituting the outer and inner coversheets of the article, absorbent layers etc., which are joined together for example by thermobonding, ultrasonic welding or gluing in a manufacturing process at high speed.

Other components like elastic threads, fastening tabs, waistbands, side panels, belts etc. may also be incorporated in articles like diapers, pant diapers and incontinence guards.

All these components must be applied and fastened in a correct position of the article. Cutting operations, in which selected lengths of materials are cutoff, are also occurring in the manufacture of absorbent articles, and the cut-off has to be made in a precise location. Control and synchronization of the joining of the different components in an absorbent article, application of a substrate to a selected area of a component, control of thermobonding, welding and glue patterns as well as of cutting operations, is of critical importance for the product quality.

Document US 5,045,135 discloses a method for controlling the cut off register in a diaper machine. A diaper web having a plurality of longitudinally spaced apart absorbent pads thereon passes a detection means detecting the edge of the pad. The cut off means is controlled by the signals from the detection means.

Document EP 1 199 057 A discloses a method for manufacturing an absorbent article having a predetermined pattern arranged at a predetermined position of the article. The pattern is printed on a continuously moving web material fed at a selected speed and is cut into selected lengths at a predetermined position. The position of the printed pattern is detected and the signal is used for controlling the feeding speed of the web, thereby obtaining the printed pattern at a correct location with respect to the cut off position.

In these two documents an edge of a pad and a printed pattern respectively is visually detected and used for controlling certain process steps. However such product features which are visually detectable at a certain position that may be used for process control are not always available and it therefore exists a need for improvement of the flexibility of the process control.

Document US20090194218 offers a more flexible process control however this system is too costly as it requires three cameras and the use of thermochromic fibers which are costly.

### OBJECT AND SUMMARY

An object of the present disclosure is to provide a solution to the above problem and to provide a flexible cost-effective system for controlling and synchronizing the cutting step in the manufacturing of absorbent articles.

These and further objects have been solved by a method for measuring and detecting welding patterns and cut locations on a web of absorbent articles (100), preferably for detecting if a web of absorbent articles (100) has been correctly cut into discrete absorbent articles (1), said method comprising the steps of:
a) bonding at least two substrate layers, preferably with elastic material in between, according to a welding pattern (50) said welding pattern (50) comprising a first welding sub-pattern (50A), a second welding sub-pattern (50B) and a gap (51) in between said first and second welding sub-pattern (50A,50B) ;
b) cutting said at least two substrate layers within the welding pattern (50) to separate said web (100) and form discrete absorbent articles (1);
c) collecting with visual detecting means (72) an image of the welding pattern (50) to determine the location of the cut made in step b) ; preferably emitting said image collected to a processing unit as an output signal;
d) processing the image collected in step c) and comparing it against a set of images and/or values, preferably said set of images and/or values being recorded beforehand in a processing unit (78); and
e) if said collected and processed image deviates from the set of images and/or values, sending a signal to a control unit (80) which takes corrective measures.

By "correctly cut" it is meant that the cut location is located in a rightful or intended position meaning between the sub-welding patterns 50A,50B or in or within the gap 51.

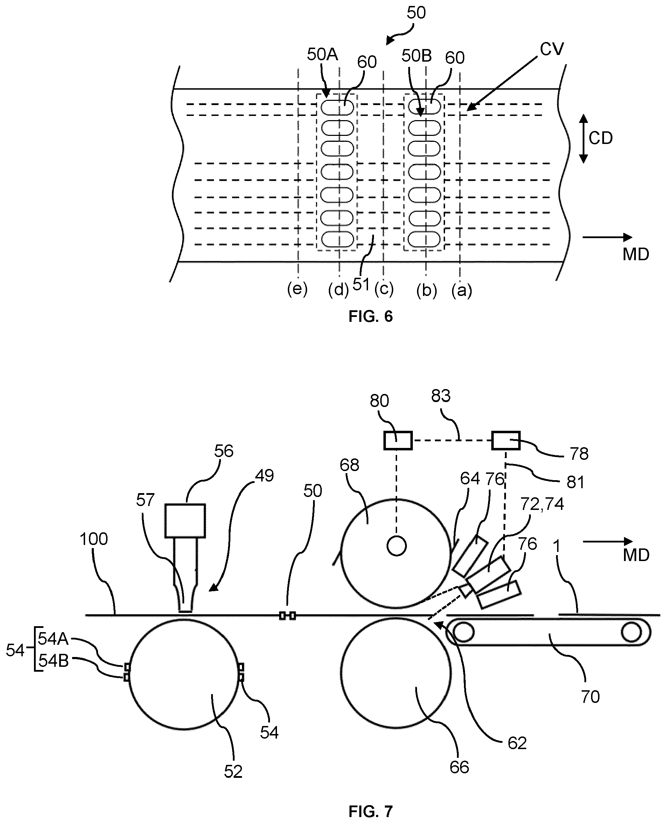

According to an embodiment, said corrective measures comprise :
a) controlling a welding device (57) parameters such as energy input or positioning of the device;
b) controlling positioning, web speed and/or web tension of the web (100) materials;
c) controlling and synchronizing a cutting device (68) in response to the location of the welding pattern (50) as illustrated in FIG. 7;
d) triggering an alarm signal to stop the process line or indicate that measures need to be taken in the process control; and/or
e) isolate the absorbent articles (1) with cuts with erroneous location.

According to an embodiment, the data is submitted from said visual detecting means (72) to a processing unit (78) and then to a control unit (80) for control of a process step and/or a process parameter; said process step and/or process parameter being controlled in response to the data submitted from the visual detecting means (72).

According to an embodiment, the position of the cut is controlled by comparing said cut with a set image and/or value and/or detected location of a component of said article, said control element inducing adjustment of one or more process steps and/or process parameters when the position and/or configuration of said cut location deviates from said set image and/or value and/or said detected location.

According to an embodiment,
- if the cut location detected is downstream of the second welding sub-pattern (50B) with respect to the machine direction (MD), or
- if the cut location detected is within the area of the second welding sub-pattern (50B);
then the image processing unit (78) sends a signal to the control unit (80) to change a process parameter or process step, preferably accelerating the rotational speed of the cutting roller (68).

According to an embodiment,
- if the cut location detected is in the area of the first welding sub-pattern (50A), or
- If the cut location detected is upstream of the first welding sub-pattern (50A) with respect to the machine direction,
then the image processing unit (78) sends a signal to the control unit (80) to change a process parameter or process step, preferably decelerating the rotational speed of the cutting roller (68).

The disclosure also pertains to a method for assembling and manufacturing a pant-type absorbent article, said method comprising:
a. providing a topsheet;
b. providing an absorbent core;
c. optionally providing an acquisition distribution layer,
d. providing a backsheet
e. assembling said topsheet, absorbent core, optionally acquisition distribution layer and backsheet, said absorbent core and optionally acquisition distribution layer being arranged between the topsheet and backsheet thereby forming an absorbent insert;
f. providing a front panel ;
g. providing a back panel ;
h. joining said absorbent insert to said front and back panels ;
i. folding front and/or back panel and/or absorbent insert to bring them in facing relationship;
j. bonding front and back panels according to said welding pattern and;
k. cutting and detecting if said web of absorbent articles (100) has been correctly cut according to the method according to any of the claim 1 to 6.

The disclosure also pertains to an apparatus for measuring and detecting welding patterns and cut locations on a web of absorbent articles (100) according to a method described herein comprising:
- visual detecting means (72),
- a welding unit (49), and
- a cutting unit (62),
wherein the visual detecting means (72) is arranged in proximity to the cutting unit (62) in order to collect with said visual detecting means (72) an image of the welding pattern (50) to determine the location of the cut.

The terms "visual detecting means" and "image recording means" are synonymous and can be interchanged.

According to an embodiment, the visual detecting means (72) comprises a charge-coupled device image sensor or a charge-coupled device camera (74)

According to an embodiment, the visual detecting means (72) are arranged downstream of the cutting unit (62) with respect to the machine direction (MD).

According to an embodiment, the visual detecting means (72) is coupled with two lighting systems (76), said lighting systems (76) being arranged in proximity to the cutting unit (62).

According to an embodiment, the apparatus further comprises a processing unit (78) to process the image collected by the visual detecting means (72), said processing unit (78) being in electronic communication with the visual detecting means (72).

### BRIEF DESCRIPTION OF DRAWINGS

The disclosure will be described in the following in greater detail by way of examples and with reference to the accompanying drawings, in which:
Fig. 1 is a perspective view showing an embodiment of a disposable absorbent article ;
Fig. 2 is an exploded perspective view showing an absorbent article as longitudinally extending;
FIG. 3 is a top view of folded multiple absorbent articles, or a web of absorbent articles, with the front and back side panel, or the front and back waist material in a facing relationship;
FIG. 4, 5 and 6 are close-up views of FIG. 3 illustrating different welding patterns;
FIG. 7 is a schematic side view of the apparatus according to an embodiment.

### DEFINITIONS

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

The terms "joined" or "welded" or "associated" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

The inner liquid permeable cover forms the inner cover of the absorbent article and in use is placed in direct contact with the skin of the wearer. The inner liquid permeable cover can comprise a nonwoven material, e.g. spunbond, meltblown, carded, hydroentangled, wetlaid etc. Suitable nonwoven materials can be composed of natural fibres, such as woodpulp or cotton fibres, manmade fibres, such as polyester, polyethylene, polypropylene, viscose etc. or from a mixture of natural and manmade fibres. The inner liquid permeable cover material may further be composed of tow fibres, which may be bonded to each other in a bonding pattern. Further examples of inner liquid permeable cover materials are porous foams, apertured plastic films, laminates of film nonwovens etc. The materials suited as inner liquid permeable cover materials should be soft and non-irritating to the skin and be readily penetrated by body fluid, e.g. urine or menstrual fluid. The inner liquid permeable cover may further be different in different parts of the absorbent article.

The outer liquid impermeable cover forms the outer cover of the absorbent article at least on the core area thereof. The outer liquid impermeable cover can comprise a thin plastic film, e g a polyethylene or polypropylene film, a nonwoven material coated with a liquid impervious material, a hydrophobic nonwoven material, which resists liquid penetration, or a laminate, e.g. of a plastic film and a nonwoven material. The outer liquid impermeable cover material maybe breathable so as to allow vapour to escape from the absorbent structure, while still preventing liquids from passing through. Examples of breathable outer liquid impermeable cover materials are porous polymeric films, nonwoven laminates from spunbond and meltblown layers, laminates from porous polymeric films and nonwoven materials.

The "absorbent structure" or "absorbent core" or "absorbent insert" is the absorbent structure disposed between the two covers of the absorbent article. The absorbent structure can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent structure.

It is conventional in absorbent articles to have absorbent structures comprising layers of different properties with respect to liquid receiving capacity, liquid distribution capacity and storage capacity. The thin absorbent bodies, which are common in for example baby diapers and incontinence guards, often comprise a compressed mixed or layered structure of cellulosic fluff pulp and superabsorbent polymers. The size and absorbent capacity of the absorbent structure may be varied to be suited for different uses such as for infants or for adult incontinent persons.

A so called acquisition layer may be arranged between the inner liquid permeable cover and the absorbent structure. The acquisition layer is adapted to quickly receive and temporarily store discharged liquid before it is absorbed by the underlying absorbent structure. Such acquisition distribution layers are well known in the art and may be composed of porous fibrous waddings or foam materials.

### DESCRIPTION OF PREFERRED EMBODIMENTS

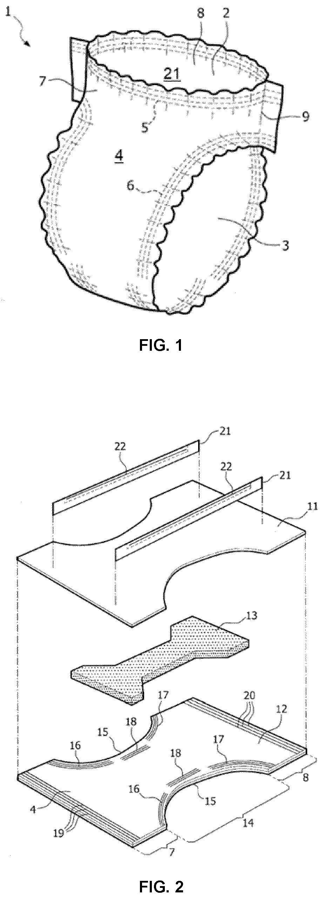

Referring to FIG. 1, an absorbent article 1 in the form of pants type and has a waist-hole 2 and a pair of leg-holes 3 formed on a textile-like outer cover 4. In an embodiment, this outer cover 4 is a laminate of materials providing a soft and tactile appearance. The waist-hole 2 is circumferentially provided with a waist surrounding elastic member 5 and the leg-holes 3 are also circumferentially provided with leg surrounding elastic members 6, respectively. Front and rear bodies defining front and rear sections, or regions, 7, 8 of the pants 1, or pant diaper 1, lie one upon another along transversely opposite side edges and are integrally bonded to each other by bonding lines 9 so as to form side seams. The front and back section, or sections, 7,8 are joined along said side edges respectively, for example by adhesive, ultrasonic welding, heat sealing or the like, so as to form side seams.

In another embodiment, the pant diaper 1 comprises a front region, a back region and a crotch region between the front region and back region, the pant diaper 1 comprises a liquid pervious inner cover, a liquid impervious outer cover and an absorbent core 7 partially or fully disposed between the inner and outer covers. The pant diaper is intended to enclose the lower part of the wearer's trunk like a pair of absorbent pants. The front and back regions may have different material composition than the crotch region, for example only the crotch region may contain liquid impermeable material. The front and back regions and are joined to each other along two opposite side edges respectively, to define a waist-opening and a pair of leg openings. The front and back regions, or sections, are joined along said side edges respectively, for example by adhesive, ultrasonic welding, heat sealing or the like, so as to form side seams. The front and back regions can either be joined along their side edges with the inner cover facing inwards in the side seams, as is shown in the drawings. Alternatively they are joined in an overlapped manner with the inner cover of either the front or back region facing the outer cover of the opposite region.

In other embodiment, the absorbent article 1 comprises an absorbent insert comprising a liquid permeable topsheet, a liquid impermeable backsheet and an absorbent core and optionally an acquisition distribution layer arranged between topsheet and backsheet. The absorbent insert is deposited onto a front panel and back panel which are discrete panels. The front panel and back panel comprises a laminate of nonwoven layers and elastic material. For example both the front and back panel comprise a outer cover an inner cover and elastic material arranged between the outer cover and inner cover. A portion of the inner and/or outer covers can be folded over the longitudinal extremities of the absorbent insert.

Referring to FIG. 2 which illustrates an example of embodiment, the absorbent article 1 comprises, as viewed in the direction of thickness, a laminate outer wrap 4 including a liquid-permeable topsheet 11, a liquid-impermeable backsheet 12 and a liquid-absorbent core 13 disposed between these two sheets 11, 12. Portions of the topsheet and backsheet 11, 12 extending outward beyond a peripheral edge of the core 13 are water-tightly bonded together and the core 13 is intermittently or fully bonded to at least one of the sheets 11, 12. The absorbent article 1 is longitudinally composed of the front and rear sections 7, 8 and a crotch section 14 interposed there between. The crotch section 14 is formed along transversely opposite sides with a pair of circular-arc shaped notches destined to define leg surrounding edges 15, respectively. First (front-half), second (rear-half) and third (core shaping) elastic members 16,17 and 18 are attached to the inner surface of the backsheet 12 facing the topsheet 11 in association with the respective leg-hole defining edges 15. Of the first, second and third elastic members 16,17 and 18 each comprising single or plural elastic strings, the first and second elastic members 16, 17 are attached to the backsheet 12 with a tension directed at least partly along a pair of leg-hole defining edges 15 and the third elastic members 18 are attached to the backsheet 12 with a tension at least partly longitudinally of the diaper 1 and allowing for easy cup formation so as to act as core shaping elastic members. Fourth elastic members 19 and 20 are attached to the inner surface of the backsheet 12 facing the topsheet 11 in association with the respective edges defining waist opening 4. As additional protection against the sideways leakage of bodily fluids or loose faecal material, a pair of upstanding cuffs 21 containing fifth elastic members 22 is attached to the topsheet 11.

In other words, the waist area and at least a part of the leg opening area are elasticized. The elastification is usually accomplished by a plurality of elastic members, such as elastic threads 19,20, contractably affixed to a material layer or between material layers. The elastification of the waist area is according to the embodiment shown in the drawings accomplished by an elastic waist band comprising a substantially non-elastic nonwoven material that is elasticized by elongate elastic members 19,20, such as elastic threads, contractably affixed inside the waist band.

The absorbent core 13 may be any means which is generally compressible, conformable, non-irritating to the wearer's skin, and capable of absorbing and retaining liquids and certain body exudates. A preferred absorbent core 13 has a garment surface and a body surface and comprises an absorbent layer. The absorbent layer may be manufactured in a wide variety of sizes and shapes (e.g., rectangular, hourglass, I-shaped, T-shaped, etc.). It may be preferred that the absorbent core be narrower in the crotch region. The absorbent layer may be manufactured from a wide variety of liquid absorbent materials commonly used in disposable diapers and other absorbent articles, such as softwood or hardwood pulp. Examples of other suitable absorbent materials include creped cellulose wadding, absorbent foams, cellulosic acetate fibers, absorbent sponges, super absorbent polymers, absorbent gelling materials, or any equivalent materials or combination of materials. The total absorbent capacity of the absorbent layer should, however, be compatible with the design exudate loading in the intended use of the absorbent article 1. Further, the size and absorbent capacity of the absorbent core 13 may be varied to accommodate wearers ranging from infants through adults.

The absorbent core 13 may include a matrix of hydrophilic fibres, such as a web of cellulosic fibres, mixed with particles, fibres and/or a compound of a high-absorbency material such as that commonly known as super absorbent particles. The term "high-absorbency material" refers to materials that are capable of absorbing at least 10 times their own weight in liquid. The absorbent core may comprise, preferably consist of, super absorbent hydrogel-forming particles and/or wood pulp fluff. The wood pulp fluff may be exchanged with synthetic, polymeric, melt-blown fibres or with a combination of melt-blown fibres and natural fibres. The high-absorbency material may be substantially homogeneously mixed with the hydrophilic fibres or may be non-uniformly mixed.

The absorbent article 1 extends in a longitudinal direction and a transverse direction.

The absorbent articles 1 in the form of a pant diaper or incontinence pant shown in the drawings is only one example of a personal care absorbent article and the embodiments of the disclosure may be applied to any type of absorbent article falling under the definition given above, including so called open diapers in which the front and back portions are fastened together by fasteners, such as adhesive tape or hook-and-loop type fasteners, belted diapers wherein belt members are used to attach the article around the waist of the wearer, incontinence pads, sanitary napkins and panty liners intended to be worn in the panties of the wearer etc.

One purpose may be to control one or more process steps and/or process parameters.

Another purpose may be to generate an error or alarm signal when a detected value deviates from a set value, indicating that there is something in the manufacturing process that needs to be corrected and eventually send the absorbent articles with deviated values to scrap. Below an example will be described of parts of a manufacturing process for manufacturing a pant diaper according to the embodiment shown in FIGS. 1 and 2.

Another purpose may be to adapt the process line to send the article to scrap to when a detected value deviates from a set value, indicating that there is something in the manufacturing process that needs to be corrected. Below an example will be described of parts of a manufacturing process for manufacturing a pant diaper according to the embodiment shown in FIGS. 1 and 2.

The pant diaper 1 may be manufactured by assembling the different components of the absorbent article 1 on a conveying unit. Once the absorbent insert is arranged within the inner and/or outer covers, or on the front and back panels the web of absorbent articles 100 is first welded by a welding unit 49 to form the side seams and then cut to separate the web of absorbent articles into individual or discrete absorbent articles.

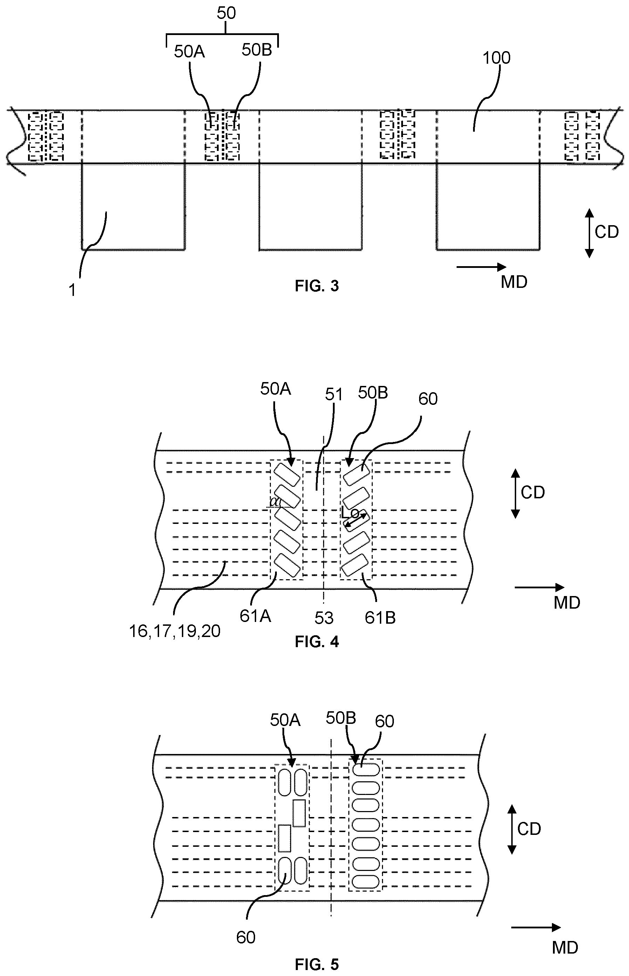

The welding unit 49 creates bonds and bonds the inner and outer covers together. The welding unit 49 creates bonds according to a welding pattern 50 comprising two welding sub-patterns 50A, 50B as illustrated in FIG. 3. As illustrated in FIG. 7, the welding unit 49 comprises an embossed roller 52, or an embossing roller 52, for example an anvil roller 52 comprising on its outer surface at least two sub-pattern imprinting surfaces 54, wherein a first pattern imprinting surface 54A is adapted to form a first bond according to a first welding sub-pattern 50A, and a second pattern imprinting surface 54B is adapted to form a second bond according to a second welding sub-pattern 50B. The welding unit 49 further comprises a welding device 56 such as a ultrasonic welding device or a heated compressing roller, the welding device 56 and the embossed roller 52 forming a nip where the web of absorbent article 100 passes through or in-between. The web of absorbent articles 100, meaning the inner and outer covers, passes through the nip and are bonded according to the first and second welding sub-patterns 50A,50B. According to an embodiment, the ultrasonic welding device 56 comprises a vibration generator, for example and not limited to, a magnetostrictive or piezoelectric transducer, said generator being attached to a tapering rod or probe usually made out of metal, such as and not limited to titanium, aluminum or steel, usually called a sonotrode 57. The sonotrode 57 is submitted to the vibrations generated by the generator and said sonotrode then applies this vibrational energy to the combined first and second waist belt material, meaning the inner and/or outer covers. The vibrational energy causes the partial melting of the web material so as to bond the first and second web material in these melting points. The melting points, which can be considered as bonding regions are defined by the embossment patterns 54, or sub-pattern imprinting surfaces 54, of the embossment roller 52.

The welding pattern 50 comprises a first welding pattern 50A a second welding pattern 50B and a gap 51 in between said first and second welding sub-patterns 50A,50B. In other words, the welding patterns are separated by a gap 51 meaning a non-welded or a non-bonded area where the two layers or covers and/or elastic are not bonded. The first and second welding sub-patterns 50A,50B can be identical (FIG. 6) or different in shape and/or dimensions and/or with different elongated elements 60 (FIG. 4 or 5). The gap is comprised between 0.1 and 5 mm, preferably between 0.1 and 2 mm, more preferably between 0.1 and 0.9 mm.

The bonding regions forming a welding pattern 50, which comprise, preferably consists of, elongated elements 60. More specifically, the sub-pattern 50A,50B each comprises a at least one, preferably a plurality of elongated elements 60 arranged in an area 61A,61B. More particularly, the elongated elements corresponds to depressions, where the inner and outer covers and eventually the elastics elements 19,20 are compressed and/or heated together. By applying heat and/or pressure onto the inner and outer covers and eventually the elastics elements 19,20 bonding regions are formed according to said welding pattern 50. The elongated elements 60 can be elongated rectangular, e.g. FIG. 4 or 5 or oblong elements, e.g. FIG. 5, extending in the longitudinal direction or in the transversal direction, meaning in the machine direction MD or in the cross direction CD. Alternatively or in combination, the elongated elements can be elongated oblique elements 60, e.g. FIG. 4, having an angle α respective to the longitudinal axis or machine direction MD, wherein the angle α is greater than 0 ° and less than 90 °, preferably from 15 ° to 75 °, more preferably from 20 ° to 70 °, and preferably wherein at least one, preferably at least 10%, more preferably at least 40%, even more preferably at least 50%. Preferably substantially all of the elongate elements have an overall length (Lo) which is comprised between 1 and 20 mm, preferably between 2 and 10 mm. The sealing pattern, meaning the welding pattern 50, has an impact on the size of the side seams, smaller welding patterns imply smaller side seams. It is preferably to have smaller side seams to save on raw material and so that the absorbent article 1 is more discreet for the wearer. The welding pattern can comprise a combination of longitudinally/vertically extending elements 60 and oblique extending elements 60.

Once the outer and inner cover are joined or bonded, the web of absorbent article 100 is then conveyed by a conveying unit such as a conveyor belt or by conveying rollers, to a cutting unit 62 comprising a cutting device 68 with a cutting knife 64 or blade, for example arranged on a rotating roller and a an anvil roller 66. The cutting device 68, or cutting knife roller 68 and the anvil roller 66 define a nip where the welded web of absorbent article 100 passes through, or in between. The knife 64 then cuts and separates the web of absorbent articles 100 into individual, separate or discrete, absorbent articles 1 and forms said individual or separate absorbent articles 1. The knife 64 cuts the web of absorbent articles 100 at a cut location 53, see FIG. 4. The cut location 53 is preferably arranged or located or position between the two sub-pattern 50A,50B within the gap 51. The apparatus can comprise an additional conveying device 70, such as a conveyor belt, rotating at a faster speed than the web of absorbent article 100 in order to increase the gap between the separated, i.e. cut, absorbent articles 1.

According to the present disclosure the apparatus comprises a visual detecting means 72 arranged in proximity of or to the cutting unit 62, preferably downstream of the cutting unit 62 with respect to the machine direction MD, to detect where the cut has been made, meaning to detect the cut location. By "arranged in proximity" it is meant that the visual detecting means 72 are arranged, or positioned, near the cutting unit 62 in a way that they can effectively detect and measure the cutting edge of the knife and absorbent article and see a contrast in light reflection and collect such image. As used herein, "in proximity" means within the minimum physical range for detection and image collection of the welding pattern and/or cut location. In other words, "in proximity" means that the visual detecting means 72 are able to detect and collect an image of the absorbent article 1, namely of the welding pattern 50 at the side seams, at the cutting unit 62, meaning when the absorbent article 1 or web 100 is within the cutting unit 62.

Indeed, welding or bonding the inner and/or outer covers and/or waist band material according to the welding pattern 50 comprising the first and second welding sub-pattern 50A,50B results in a change of appearance and/or a change of contrast. Namely the portions that are bonded and the area that are unbonded will reflect light differently hence it is possible using a light sensible camera to detect the welding patterns 50A,50B. In other words, the first and second welding sub-patterns 50A,50B are contrasting from the surrounding parts of the material web and thus are easily detectable by visual detecting means or image recording means 72. The apparatus, for example comprises an automated camera 74 inspection system such as a charge-coupled device (CCD) image sensor 74 or a CCD camera 74 coupled, or combined, with at least one, preferably two, lighting systems 76 such as high-intensity lights 76. As illustrated in FIG. 7, when there is two lighting devices 76, there are arranged on each side of the camera 74, or in other words, the camera 74 is arranged between the lighting devices 76. Preferably, said lighting systems 76 are arranged in proximity to the cutting unit 62, meaning that the lighting devices 76 are positioned next to the visual detecting means 72 to enable the visual detecting means 72 to detect and collect an image of the absorbent article 1, namely of the welding pattern 50 at the side seams, at the cutting unit 62, meaning when the absorbent article 1 or web 100 is within the cutting unit 62. In other words, the lighting device(s) 76 are positioned in a way to illuminate the cutting unit 62.

In the illustrated embodiments the welding sub-patterns 50A,50B are made in the waist band area meaning in the front and back panels, namely in the side seams, and are detected by said image recording means 72, for example in the form of a so called Vision-system, which is a system for automatic inspection, for example a CCD camera 74 or a digital double-speed colour camera as mentioned hereabove. The vision-system is preferably coupled with a lighting system 76, such as having two high-intensity lights, e.g. High-Intensity Discharge Lamps (HID Lamps).

At least one image of the welding sub-pattern(s) 50A,50B is recorded beforehand in a processing unit 78. The image recording means 72 develops a video signal 81 that is delivered to an image digitizer and analysing unit 78 or an image processing unit 78, which compares the recorded image against a set of previously established images. If the recorded image deviates from the established value range (set value) a signal 83 is sent to a control unit 80, which may have one or more of the following functions:
a) Controlling the ultrasonic welding device 57 for example with respect to energy input, positioning of the device etc.
b) Controlling positioning, web speed and/or web tension of the web materials introduced in the ultrasonic welding device;
c) Controlling and synchronizing the cutting device 68 in response to the location of the welding pattern as illustrated in FIG. 7;
d) Triggering an alarm signal 34, 35 to indicate that measures need to be taken in the process control; and/or
e) isolate the absorbent articles 1 with erroneous cuts and send for scrapping to be recycled or thrown away, i.e. reject the absorbent articles 1 with erroneous cuts meaning absorbent articles 1 where the cut location is erroneous or located in a wrongful position i.e. not between the sub-welding patterns 50A,50B or not in the gap 51.

In the last mentioned case the measures could be to check whether an equipment, for example an ultrasonic horn or pattern roll is worn out and needs to be replaced, or to check whether one or more process steps or parameters, such as temperature, web speed, web tension, positioning of equipment or web materials need to be adjusted.

According to an embodiment, the processing can be as following:
- the image recording means 72, or camera 74, collects an image of a welding pattern and cut;
- it sends it to the processing unit 78 which creates a virtual line or a virtual area for the welding pattern and sub-patterns and a virtual line for the cut position based on the set of images and/or values recorded beforehand;
- the processing unit 78 measure the distance between the virtual line or area for the welding pattern and the virtual line for the cut position;
- if the distance between said virtual lines is too great then send an order to the control unit 80 to alter a process parameter or step such as synchronous positioning movement of the knife and anvil roller.

Naturally, the different unit such as the cutting unit 62, the control unit 80 or the processing unit 78 comprise the adapted motion systems such as motion controllers, servo motors to carry out the changes in process parameter or step.

The embodiment illustrated in FIG. 6 shows the different deviation from the established value range (set value). The welding pattern 50 comprises here two identical welding sub-patterns 50A,50B with transversally extending, or MD extending, elongated elements 60. The arrow CV illustrates the Camera View (CV), the camera 74 can save images of the welding pattern 50 and send them to the image processing unit 78. There are different scenarios depending on the position where the cutting unit 62 has made a cut or in other words depending on where the cut location is, the scenarios are as following :
(a) if the cut is made in the vicinity of line (a), meaning downstream of the second welding sub-pattern 50B with respect to the machine direction, the camera 74 will not detect any welding pattern 50 or any welding sub-pattern 50A,B ; or
(b) if the cut is made in the vicinity of line (b), meaning in the area of the second welding sub-pattern 50B, the camera 74 will detect an irregularly-shaped sub-pattern.

In scenarios (a) and (b), the image processing unit 78 sends a signal 83 to the control unit 80 to change a parameter, for example accelerating the rotational speed of the cutting roller 68 so that the web 100 can be cut more upstream.
(c) The cut is made in the gap 51 between the two welding sub-pattern 50A,50B.

In scenario (c), the process is functioning as it should and there is no correction to undertake.
(d) if the cut is made in the vicinity of line (d), meaning in the area of the first welding sub-pattern 50A, the camera 74 will detect an irregularly-shaped sub-pattern.
(e) If the cut is made in the vicinity of line (e), meaning upstream of the first welding sub-pattern 50A with respect to the machine direction, the camera 74 will detect two welding sub-patterns 50A,B.

In scenarios (d) and (e), the image processing unit 78 sends a signal 83 to the control unit 80 to change a parameter, for example decelerating the rotational speed of the cutting roller 68 so that the web 100 can be cut more downstream.

According to an embodiment, the visual detecting means i.e. image recording means 72 can further comprises a control system that manages the data outputs of and emits output data or a signal 81 to an image processing unit 78. The image processing unit 78 can include a processor and memory. The processor can perform various computing functions, such as positioning values and plotting said values against a collection of image recorded before-hand and also plotting said values against a time measured by an internal clock to identify which absorbent article 1 needs to be rejected or sent to scrap. The image processor preferably include a dedicated video processor for image processing. The signals 81,83 between the image recording means 72 and the image processing unit 78 and the control unit 80 are preferably electronic signals for example communicated via cables, wireless or Bluetooth technologies.

The camera 74 and vision system is preferably arranged downstream of the cutting knife. However the same principle can be used if it is placed upstream of the cutting unit.

The present disclosure also pertains to a method for assembling and manufacturing a pant-type absorbent article, said method comprising:
a) providing a topsheet;
b) providing an absorbent core;
c) optionally providing an acquisition distribution layer,
d) providing a backsheet
e) assembling said topsheet, absorbent core, optionally acquisition distribution layer and backsheet, said absorbent core and optionally acquisition distribution layer being arranged between the topsheet and backsheet thereby forming an absorbent insert;
f) providing a front panel ;
g) providing a back panel ;
h) joining said absorbent insert to said front and back panels ;
i) folding front and/or back panel to bring them in facing relationship;
j) bonding front and back panels according to said welding pattern and;
k) cutting and detecting if said web of absorbent articles (100) has been correctly cut according to the method disclosed herein.

Naturally, the method for assembling and manufacturing a pant-type absorbent article can further comprise a step of adjusting a parameter such as accelerating of decelerating the cutting knife roller depending on the image detected in step k).

## Claims

1. Method for measuring and detecting welding patterns and cut locations on a web of absorbent articles (100), said method comprising the steps of:
a) bonding at least two substrate layers according to a welding pattern (50) said welding pattern (50) comprising a first welding sub-pattern (50A), a second welding sub-pattern (50B) and a gap (51) in between said first and second welding sub-pattern (54A,54B) ;
b) cutting said at least two substrate layers within the welding pattern (50) to separate said web (100) and form discrete absorbent articles (1);
c) collecting with visual detecting means (72) being arranged in proximity to a cutting unit (62) downstream of the cutting unit (62) an image of the welding pattern (50) to determine the location of the cut made in step b) ;
d) processing the image collected in step c) and comparing it against a set of images and/or values, preferably recorded beforehand in a processing unit (78); and
e) if said collected and processed image deviates from the set of images and/or values, sending a signal to a control unit (80) which takes corrective measures.

2. Method according to claim 1, wherein said corrective measures comprise :
a) controlling a welding device (57) parameters such as energy input or positioning of the device;
b) controlling positioning, web speed and/or web tension of the web (100) materials;
c) controlling and synchronizing a cutting device (68) in response to the location of the welding pattern (50) ;
d) triggering an alarm signal to stop the process line or indicate that measures need to be taken in the process control; and/or
e) isolate the absorbent articles (1) with cuts with erroneous location.

3. Method according to claim 1 or 2, wherein the data is submitted from said visual detecting means (72) to a processing unit (78) and then to a control unit (80) for control of a process step and/or a process parameter; said process step and/or process parameter being controlled in response to the data submitted from the visual detecting means (72).

4. Method according to any of the claims 1 to 3, wherein the position of the cut is controlled by comparing said cut with a set image and/or value and/or detected location of a component of said article, said control element inducing adjustment of one or more process steps and/or process parameters when the position and/or configuration of said cut location deviates from said set image and/or value and/or said detected location.

5. Method according to any of the preceding claims, wherein
- if the cut location detected is downstream of the second welding sub-pattern (50B) with respect to the machine direction (MD), or
- if the cut location detected is within the area of the second welding sub-pattern (50B);
then the image processing unit (78) sends a signal to the control unit (80) to change a process parameter or process step, preferably accelerating the rotational speed of the cutting roller (68).

6. Method according to any of the preceding claims, wherein
- if the cut location detected is in the area of the first welding sub-pattern (50A), or
- If the cut location detected is upstream of the first welding sub-pattern (50A) with respect to the machine direction,
then the image processing unit (78) sends a signal to the control unit (80) to change a process parameter or process step, preferably decelerating the rotational speed of the cutting roller (68).

7. Method for assembling and manufacturing a pant-type absorbent article, said method comprising:
a) providing a topsheet;
b) providing an absorbent core;
c) optionally providing an acquisition distribution layer,
d) providing a backsheet
e) assembling said topsheet, absorbent core, optionally acquisition distribution layer and backsheet, said absorbent core and optionally acquisition distribution layer being arranged between the topsheet and backsheet thereby forming an absorbent insert;
f) providing a front panel ;
g) providing a back panel ;
h) joining said absorbent insert to said front and back panels ;
i) folding front and/or back panel and/or absorbent insert to bring them in facing relationship;
j) bonding front and back panels according to said welding pattern and;
k) cutting and detecting if said web of absorbent articles (100) has been correctly cut according to the method according to any of the claim 1 to 6.

8. Apparatus for measuring and detecting welding patterns and cut locations on a web of absorbent articles (100) according to a method according to any claim 1 to 6 comprising:
- visual detecting means (72)
- a welding unit (49), and
- a cutting unit (62),
wherein the visual detecting means (72) is arranged in proximity to the cutting unit (62) in order to collect with said visual detecting means (72) an image of the welding pattern (50) to determine the location of the cut, wherein the visual detecting means (72) are arranged downstream of the cutting unit (62) with respect to the machine direction (MD).

9. Apparatus according to claim 8, wherein the visual detecting means (72) comprises a charge-coupled device image sensor or a charge-coupled device camera (74)

10. Apparatus according to any of the claims 8 to 9, wherein the visual detecting means (72) is coupled with two lighting systems (76), said lighting systems (76) being arranged in proximity to the cutting unit (62).

11. Apparatus according to any of the claims 8 to 9, wherein the apparatus further comprises a processing unit (78) to process the image collected by the visual detecting means (72), said processing unit (78) being in electronic communication with the visual detecting means (72).

## Patentansprüche

1. Verfahren zum Messen und Erkennen von Schweißmustern und Schnittstellen auf einer Bahn aus saugfähigen Artikeln (100), das Verfahren umfassend die Schritte:
a) Verbinden von mindestens zwei Substratschichten gemäß einem Schweißmuster (50), das Schweißmuster (50) umfassend ein erstes Schweißuntermuster (50A), ein zweites Schweißuntermuster (50B) und einen Spalt (51) zwischen dem ersten und dem zweiten Schweißuntermuster (54A, 54B);
b) Schneiden der mindestens zwei Substratschichten innerhalb des Schweißmusters (50), um die Bahn (100) zu trennen und einzelne saugfähige Artikel (1) auszubilden;
c) Aufnehmen eines Bildes des Schweißmusters (50) mit einem optischen Erkennungsmittel (72), das in Nähe einer Schneideinheit (62) stromabwärts der Schneideinheit (62) angeordnet ist, um die Stelle des in Schritt b) vorgenommenen Schnitts zu bestimmen;
d) Verarbeiten des in Schritt c) aufgenommenen Bildes und Vergleichen davon mit einem Satz von Bildern und/oder Werten, die vorzugsweise zuvor in einer Verarbeitungseinheit (78) aufgezeichnet wurden; und
e) falls das aufgenommene und verarbeitete Bild von dem Satz von Bildern und/oder Werten abweicht, Senden eines Signals an eine Steuereinheit (80), die Korrekturmaßnahmen ergreift.

2. Verfahren nach Anspruch 1, wobei die Korrekturmaßnahmen umfassen:
a) Steuern von Parametern einer Schweißvorrichtung (57), wie Energiezufuhr oder Positionierung der Vorrichtung;
b) Steuern der Positionierung, Bahngeschwindigkeit und/oder Bahnspannung der Bahnmaterialien (100);
c) Steuern und Synchronisieren einer Schneidevorrichtung (68) als Reaktion auf die Stelle des Schweißmusters (50);
d) Auslösen eines Alarmsignals, um die Prozesslinie zu stoppen oder anzuzeigen, dass Maßnahmen in der Prozesssteuerung ergriffen werden müssen; und/oder
e) Isolieren der saugfähigen Artikel (1) mit Schnitten an fehlerhafter Stelle.

3. Verfahren nach Anspruch 1 oder 2, wobei die Daten von dem optischen Erkennungsmittel (72) an eine Verarbeitungseinheit (78) und dann an eine Steuereinheit (80) für die Steuerung eines Prozessschritts und/oder eines Prozessparameters übermittelt wird; wobei der Prozessschritt und/oder Prozessparameter als Reaktion auf die von dem optischen Erkennungsmittel (72) übermittelten Daten gesteuert wird.

4. Verfahren nach einem der Ansprüche 1 bis 3, wobei die Position des Schnitts durch Vergleichen des Schnitts mit einem festgelegten Bild und/oder Wert und/oder einer erkannten Stelle einer Komponente des Artikels gesteuert wird, wobei das Steuerelement eine Anpassung eines oder mehrerer Prozessschritte und/oder Prozessparameter veranlasst, wenn die Position und/oder Konfiguration der Schnittstelle von dem festgelegten Bild und/oder Wert und/oder der erkannten Stelle abweicht.

5. Verfahren nach einem der vorstehenden Ansprüche, wobei
- falls die erkannte Schnittstelle in Bezug auf die Maschinenrichtung (MD) stromabwärts des zweiten Schweißuntermusters (50B) liegt, oder
- falls die erkannte Schnittstelle innerhalb des Bereichs des zweiten Schweißuntermusters (50B) liegt;
die Bildverarbeitungseinheit (78) dann ein Signal an die Steuereinheit (80) sendet, um einen Prozessparameter oder Prozessschritt zu ändern, was vorzugsweise die Rotationsgeschwindigkeit der Schneidwalze (68) beschleunigt.

6. Verfahren nach einem der vorstehenden Ansprüche, wobei
- falls die erkannte Schnittstelle in dem Bereich des ersten Schweißteilmusters (50A) liegt, oder
- falls die erkannte Schnittstelle in Bezug auf die Maschinenrichtung stromaufwärts des ersten Schweißteilmusters (50A) liegt,
die Bildverarbeitungseinheit (78) dann ein Signal an die Steuereinheit (80) sendet, um einen Prozessparameter oder Prozessschritt zu ändern, was vorzugsweise die Rotationsgeschwindigkeit der Schneidwalze (68) entschleunigt.

7. Verfahren zum Zusammensetzen und Herstellen eines höschenartigen saugfähigen Artikels, das Verfahren umfassend:
a) Bereitstellen einer Oberschicht;
b) Bereitstellen eines saugfähigen Kerns;
c) optionales Bereitstellen einer Erfassungsverteilungsschicht,
d) Bereitstellen einer Unterschicht
e) Zusammensetzen der Oberschicht, des saugfähigen Kerns, optional der Erfassungsverteilungsschicht und der Unterschicht, wobei der saugfähige Kern und optional die Erfassungsverteilungsschicht zwischen der Oberschicht und der Unterschicht angeordnet sind, wobei dadurch eine saugfähige Einlage ausgebildet wird;
f) Bereitstellen einer Vorderseite;
g) Bereitstellen einer Rückseite;
h) Verknüpfen der saugfähigen Einlage mit der Vorder- und Rückseite;
i) Falten der Vorder- und/oder Rückseite und/oder der Absorptionseinlage, um sie in eine gegenüberliegende Beziehung zu bringen;
j) Verbinden der Vorder- und Rückseite gemäß dem Schweißmuster und;
k) Schneiden und Erkennen, ob die Bahn aus saugfähigen Artikeln (100) gemäß dem Verfahren nach einem der Ansprüche 1 bis 6 richtig geschnitten wurde.

8. Vorrichtung zum Messen und Erkennen von Schweißmustern und Schnittstellen auf einer Bahn aus saugfähigen Artikeln (100) gemäß einem Verfahren nach einem der Ansprüche 1 bis 6, umfassend:
- ein visuelles Erkennungsmittel (72)
- eine Schweißeinheit (49) und
- eine Schneideinheit (62),
wobei das visuellen Erkennungsmittel (72) in Nähe der Schneideinheit (62) angeordnet ist, um mit dem visuellen Erkennungsmittel (72) ein Bild des Schweißmusters (50) aufzunehmen, um die Stelle des Schnitts zu bestimmen, wobei das visuelle Erkennungsmittel (72) in Bezug auf die Maschinenrichtung (MD) stromabwärts der Schneideinheit (62) angeordnet ist.

9. Vorrichtung nach Anspruch 8, wobei das visuelle Erkennungsmittel (72) einen Charge-coupled Device-Bildsensor oder eine Charge-coupled Device-Kamera (74) umfasst.

10. Vorrichtung nach einem der Ansprüche 8 bis 9, wobei das optische Erkennungsmittel (72) mit zwei Beleuchtungssystemen (76) gekoppelt ist, wobei die Beleuchtungssysteme (76) in Nähe der Schneideinheit (62) angeordnet sind.

11. Vorrichtung nach einem der Ansprüche 8 bis 9, wobei die Vorrichtung ferner eine Verarbeitungseinheit (78) umfasst, um das Bild, das durch das visuelle Erkennungsmittel (72) aufgenommen wird, zu verarbeiten, wobei die Verarbeitungseinheit (78) in elektronischer Verbindung mit dem visuellen Erkennungsmittel (72) steht.

## Revendications

1. Procédé de mesure et de détection de motifs de soudage et d'emplacements de coupe sur une bande d'articles absorbants (100), ledit procédé comprenant les étapes consistant à :
a) lier au moins deux couches de substrat selon un motif de soudage (50), ledit motif de soudage (50) comprenant un premier sous-motif de soudage (50A), un second sous-motif de soudage (50B) et un interstice (51) entre ledit premier et ledit second sous-motif de soudage (54A, 54B) ;
b) couper lesdites au moins deux couches de substrat à l'intérieur du motif de soudage (50) afin de séparer ladite bande (100) et de former des articles absorbants discrets (1) ;
c) collecter, à l'aide d'un moyen de détection visuelle (72) disposé à proximité d'une unité de coupe (62) en aval de l'unité de coupe (62), une image du motif de soudage (50) afin de déterminer l'emplacement de la coupe effectuée à l'étape b) ;
d) traiter l'image collectée à l'étape c) et sa comparaison à un ensemble d'images et/ou de valeurs, de préférence enregistrées au préalable dans une unité de traitement (78) ; et
e) si ladite image collectée et traitée s'écarte de l'ensemble d'images et/ou de valeurs, envoyer un signal à une unité de commande (80) qui prend des mesures correctives.

2. Procédé selon la revendication 1, dans lequel lesdites mesures correctives comprennent :
a) la commande de paramètres d'un dispositif de soudage (57), tels que l'entrée d'énergie ou le positionnement du dispositif ;
b) la commande du positionnement, de la vitesse de la bande et/ou de la tension de bande des matériaux de la bande (100) ;
c) la commande et la synchronisation d'un dispositif de coupe (68) en réponse à l'emplacement du motif de soudage (50) ;
d) le déclenchement d'un signal d'alarme afin d'arrêter la ligne de processus ou d'indiquer que des mesures doivent être prises dans la commande du processus ; et/ou
e) l'isolement des articles absorbants (1) ayant des coupes dont l'emplacement est erroné.

3. Procédé selon la revendication 1 ou 2, dans lequel les données sont soumises à partir dudit moyen de détection visuelle (72) à une unité de traitement (78), puis à une unité de commande (80) pour la commande d'une étape de processus et/ou d'un paramètre de processus ; ladite étape de processus et/ou ledit paramètre de processus étant commandés en réponse aux données soumises par le moyen de détection visuelle (72).

4. Procédé selon l'une quelconque des revendications 1 à 3, dans lequel la position de la coupe est commandée par comparaison de ladite coupe à une image et/ou à une valeur définies et/ou à un emplacement détecté d'un composant dudit article, ledit élément de commande induisant l'ajustement d'un ou plusieurs étapes de processus et/ou paramètres de processus lorsque la position et/ou la configuration dudit emplacement de coupe s'écartent de ladite image et/ou valeur définies et/ou dudit emplacement détecté.

5. Procédé selon l'une quelconque des revendications précédentes, dans lequel
- si l'emplacement de coupe détecté est situé en aval du second sous-motif de soudage (50B) par rapport au sens machine (MD) ou
- si l'emplacement de coupe détecté est situé dans la zone du second sous-motif de soudage (50B) ;
alors l'unité de traitement d'image (78) envoie un signal à l'unité de commande (80) pour la modification d'un paramètre de processus ou d'une étape de processus, de préférence l'accélération de la vitesse de rotation du rouleau de coupe (68).

6. Procédé selon l'une quelconque des revendications précédentes, dans lequel
- si l'emplacement de coupe détecté est situé dans la zone du premier sous-motif de soudage (50A), ou
- si l'emplacement de coupe détecté est situé en amont du premier sous-motif de soudage (50A) par rapport au sens machine,
alors l'unité de traitement d'image (78) envoie un signal à l'unité de commande (80) pour la modification d'un paramètre de processus ou d'une étape de processus, de préférence le ralentissement de la vitesse de rotation du rouleau de coupe (68).

7. Procédé d'assemblage et de fabrication d'un article absorbant de type culotte, ledit procédé comprenant :
a) la fourniture d'une feuille supérieure ;
b) la fourniture d'un noyau absorbant ;
c) la fourniture éventuelle d'une couche de distribution d'acquisition,
d) la fourniture d'une feuille arrière
e) l'assemblage de ladite feuille supérieure, dudit noyau absorbant, éventuellement de ladite couche de distribution d'acquisition et de ladite feuille arrière, ledit noyau absorbant et éventuellement ladite couche de distribution d'acquisition étant disposés entre la feuille supérieure et la feuille arrière, de manière à former un insert absorbant ;
f) la fourniture d'un panneau avant ;
g) la fourniture d'un panneau arrière ;
h) le raccordement dudit insert absorbant auxdits panneaux avant et arrière ;
i) le pliage du panneau avant et/ou arrière et/ou de l'insert absorbant pour les mettre en face l'un de l'autre ;
j) la liaison des panneaux avant et arrière selon ledit motif de soudage et ;
k) la coupe et la détection si ladite bande d'articles absorbants (100) a été correctement coupée selon le procédé selon l'une quelconque des revendications 1 à 6.

8. Appareil de mesure et de détection de motifs de soudage et d'emplacements de coupe sur une bande d'articles absorbants (100) selon un procédé de l'une quelconque des revendications 1 à 6, comprenant :
- un moyen de détection visuelle (72)
- une unité de soudage (49) et
- une unité de coupe (62),
dans lequel le moyen de détection visuelle (72) est disposé à proximité de l'unité de coupe (62) afin de collecter, à l'aide dudit moyen de détection visuelle (72), une image du motif de soudage (50) pour la détermination de l'emplacement de la coupe, dans lequel le moyen de détection visuelle (72) est disposé en aval de l'unité de coupe (62) par rapport au sens machine (MD).

9. Appareil selon la revendication 8, dans lequel le moyen de détection visuelle (72) comprend un capteur d'image à dispositif à couplage de charge ou une caméra à dispositif à couplage de charge (74)

10. Appareil selon l'une quelconque des revendications 8 à 9, dans lequel le moyen de détection visuelle (72) est couplé à deux systèmes d'éclairage (76), lesdits systèmes d'éclairage (76) étant disposés à proximité de l'unité de coupe (62).

11. Appareil selon l'une quelconque des revendications 8 à 9, dans lequel l'appareil comprend en outre une unité de traitement (78) destinée à traiter l'image collectée par le moyen de détection visuelle (72), ladite unité de traitement (78) étant en communication électronique avec le moyen de détection visuelle (72).
